# EUROPEAN PATENT SPECIFICATION

(11) **EP 2 667 772 B1**
(45) Date of publication and mention of the grant of the patent: **13.07.2016**
(21) Application number: 12703888.3
(22) Date of filing: 19.01.2012
(51) Int. Cl.: A61B 5/0428, A61B 5/0408, A61B 5/0432

(54) **EXCHANGEABLE ELECTRODE AND ECG CABLE SNAP CONNECTOR**
AUSTAUSCHBARE ELEKTRODE UND EKG-KABEL-SCHNAPPVERBINDUNG
ÉLECTRODE REMPLAÇABLE ET CONNECTEUR À ENCLENCHEMENT DE CÂBLE ECG

(30) Priority: 27.01.2011 US 201113015173
(43) Date of publication of application: 04.12.2013
(73) Proprietor: Koninklijke Philips N.V., 5656 AE Eindhoven (NL)
(72) Inventor: CHRIST, Johannes, NL-5656 AE Eindhoven (NL); MAIER, Matthias, NL-5656 AE Eindhoven (NL)
(74) Representative: Steffen, Thomas
(86) International application number: PCT/IB2012/050265
(87) International publication number: WO 2012/101554

(56) References cited:
- WO-A1-2010/038156
- JP-A- 2001 198 096
- US-A- 6 117 077
- US-A1- 2004 077 954
- US-A1- 2005 107 714

## Description

This invention relates to a body-worn electrocardiogram (ECG) monitor assembly which includes ECG electrodes and an event recorder/monitor which is disposed on one of the electrodes. The invention further relates to an improved attachment system for the monitor assembly.

In medical care, functional modules for determining a physiological parameter of a patient, are often attached to the body of a patient. One example is a body-worn ECG monitor that is adhesively attached to a patient's skin. The monitor captures data from disposable ECG electrodes that are plugged in to the monitor. The monitor is reusable, while the ECG electrodes are disposed of for hygienic reasons.

US Patent No. 6,605,046 describes an ambulatory ECG monitor which is mounted directly to an athlete's skin. The monitor can be inserted into a pouch, which in turn is held in place by adhesive pads affixed to the skin. ECG electrodes are electrically connected to the monitor via conventional plugs. The monitor is reused, while the other components may be discarded after use.

WO 2002/2206 describes a disposable vital signs monitoring sensor band with a reusable electronics module that connects to a sensor band to enable capture of, e.g., ECG data. The module has two parts: a disposable part that contains batteries and is connected to the sensor band, and the reusable part connected to the disposable part via a clip. A track strip projection on the disposable part plugs in to a connector socket on the reusable part to establish elecrtrical connection.

Various monitoring devices are also known from documents US 2005/107714 A1, JP2001198096 A, WO 2010/038156 A1, US 2004/077954 A1, and US 117 077 A.

Because ambulatory ECG monitors are worn close to the patient's skin, it is very important to clean the monitor effectively between uses. Prior art ECG monitors such as those described above use cable connector sockets that mate with ECG electrodes. These blind hole connectors are very difficult to clean. If the ECG monitor is configured to be patient-worn over an ECG electrode having a standard snap connector, the monitor's mating socket is also difficult to clean.

Mechanical wear is also a problem in the prior art ECG monitors. Because the module is supported by the electrode snap connector, patient motion and jostling of the module can damage the electrical connection between the module and electrode within a short period of time. Such patient movement can also damage the electrode wire connectors. If the connectors are integrated with the reusable module, the entire module must be replaced, resulting in considerable expense and inconvenience.

It would therefore be desirable to overcome these problems in the prior art. What is desirable is an ambulatory ECG module which is easy to clean, is robust, and may be refurbished for reuse at lower expense.

The present invention solves the cleanliness problems inherent in the prior art. The inventive embodiments collect all hard-to-clean areas into a single, disposable component which is installed between the ECG electrodes and the ECG module. The ECG module, in turn, comprises only flush or projective electrical and support surfaces which are easy to clean.

The present invention also solves the problem of lack of robust electrical connections in prior art ambulatory ECG monitors. By locating all connections that are prone to mechanical wear and failure in a disposable component, the entire component can be easily and simply replaced prior to the next use of the ECG monitor.

In accordance with the principles of the present invention, an ambulatory ECG recorder/analyzer assembly is described having an ECG module, a base ECG electrode to which the ECG module is attached, and an exchangeable electrode and ECG cable snap connector. A plurality of ECG electrodes are electrically attached to the exchangeable electrode and ECG cable snap connector through standard lead wires and plugs.

In accordance with another aspect of the invention, a disposable interconnect for an ECG module is described having on one end a clip for attaching to the snap connector of a standard ECG electrode. The second end of the disposable interconnect comprises a plurality of electrode connector sockets to which standard ECG electrode lead wires may be plugged. One side of the disposable interconnect comprises a receiver and catch disposed to mechanically attach to a corresponding protrusion and hooks on the ECG module. The interconnect side also comprises a plurality of electrical contacts for electrical connection to the ECG module. The contacts may be spring-loaded to ensure robust contact with corresponding pins on the ECG module during use. The interconnect thus contains all of the female connection elements of the assembly. The interconnect has no expensive components, so that it may be disposed of after use.

In accordance with another aspect of the invention, a method of monitoring the ECG of an ambulatory patient is described, comprising the steps of applying disposable ECG electrodes to a patient's skin, attaching a disposable interconnect to one of the ECG electrode snap connectors to establish mechanical and electrical connection, plugging the lead wires of two additional ECG electrodes into the disposable interconnect, and attaching an ECG module to the disposable interconnect. The method is advantageous over the prior art for several reasons. First, it avoids the patient discomfort associated with pressing a prior art module directly onto an ECG electrode snap connector. It enables the quick exchange of an ECG module for another without removing electrodes or wires. And when use is complete, disassembly by reversing the above steps avoids a step of cleaning socket holes by simply disposing of the disposable interconnect instead.

In the drawings:
FIGURE 1 illustrates a prior art ECG monitor having a typical arrangement of electrodes and electrode interconnects with the body-worn recorder module.
FIGURE 2 illustrates a wearable ECG monitor assembly according to one embodiment of the present invention.
FIGURE 3a is an exploded view of the disposable interconnect and the module according to one embodiment of the present invention. FIGURE 3b shows the assembled module, interconnect, and base electrode.
FIGURE 4 illustrates a detail view of the disposable interconnect according to one embodiment of the present invention.
FIGUREs 5a, 5b, and 5c are detail views of the ECG module chassis, including the module base, electrical connect pins, and connect pins as disposed in the module base, respectively.

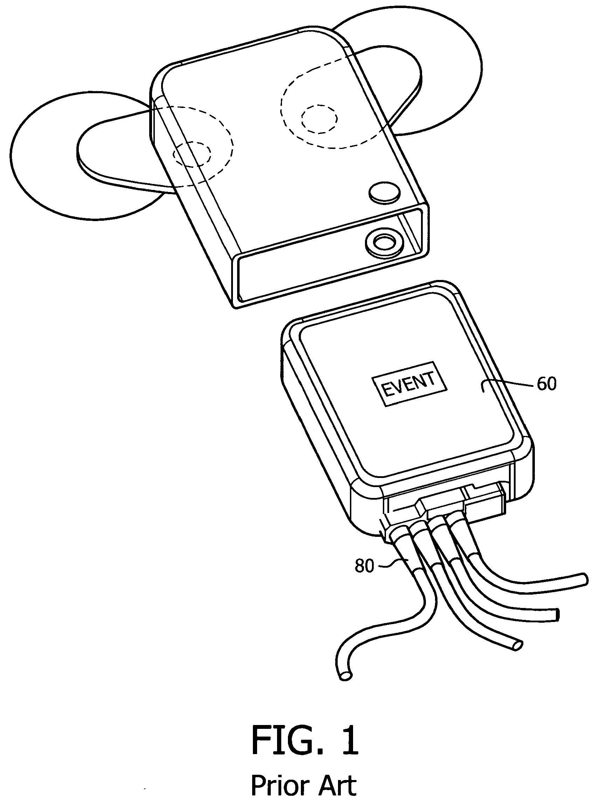

FIGURE 1 illustrates the problem solved by the present invention, as discovered by the inventors. Shown is a prior art ambulatory ECG monitor/recorder 60. As can be seen at the electrode lead wire connectors 80, monitor/recorder 60 comprises a number of connector sockets which correspond to the lead wire plugs. After use, these sockets must be decontaminated in preparation for the next use. Such cleaning is difficult. In addition, if the connector sockets become worn to the point that electrical contact with the lead wire plugs is intermittent, the entire monitor/recorder 60 must be replaced at significant expense and inconvenience.

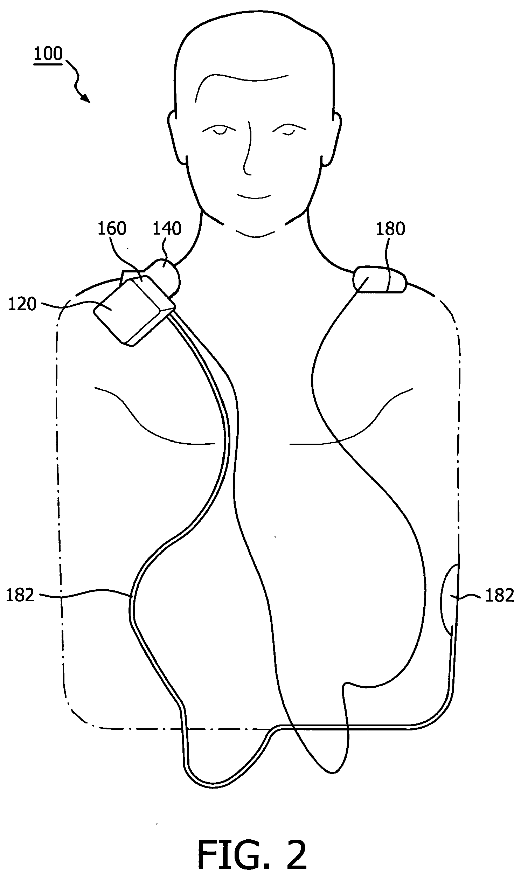

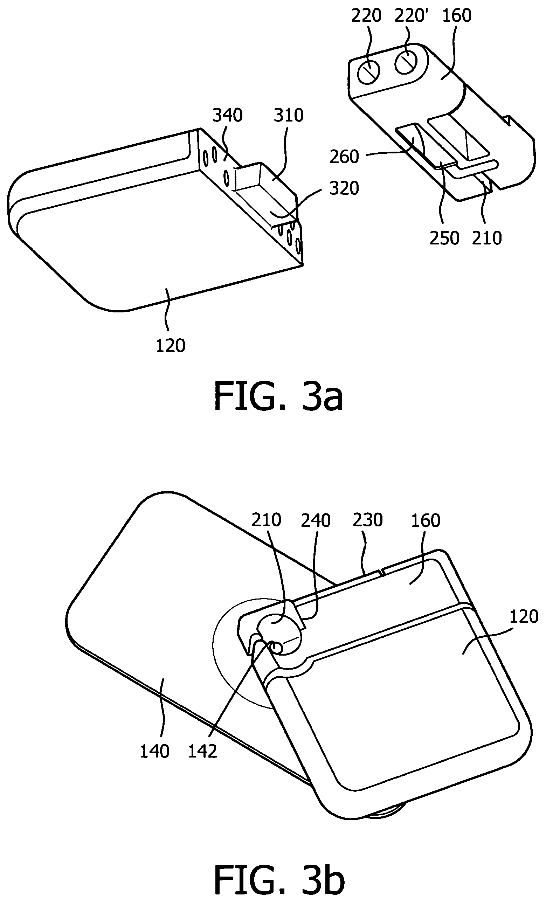

FIGURE 2 illustrates the ECG monitor assembly 100 of the present invention. The assembly comprises four components. A base ECG electrode 140 with a snap connector 142 (see FIGURE 3b) affixes the ECG monitor assembly to the patient's skin and provides an electrical contact point on the patient's body. Base ECG electrode is preferably adhered to the patient's torso at a standard location, such as near the right clavicle. The base ECG electrode may comprise a standard monitoring electrode with a snap connector, or may be a custom-designed electrode with enhanced adhesive strength for holding the assembly in place.

A remote ECG electrode 182 having a lead wire and lead wire connector comprises the second component of the ECG monitor assembly. Remote ECG electrode 182 is preferably adhered to the patient at a second standard ECG location on the patient's torso. Remote ECG electrode 182 is shown in FIGURE 2 on the patient's lower left torso, such that the line formed with base electrode 140 corresponds to a primary electro-potential axis of the patient's heart. Like base electrode 140, remote electrode 182 may be a standard monitoring electrode having a standard electrode lead wire and male connecting plug. In addition, the assembly may also include a second remote electrode 180 to sense additional ECG data.

The ECG monitor assembly also includes an ECG module 120. ECG module 120 contains at least a power source, processing circuitry, and electronic data storage for monitoring and recording ECG data obtained from base electrode 140 and remote electrodes 180 and 182. Preferably, ECG module 120 is compact enough to be worn on the patient's body, and is light enough to allow ambulatory movement of the patient while remaining securely attached.

The fourth component of the ECG monitor assembly 100 is the exchangeable electrode and ECG cable snap connector 160. Exchangeable electrode and ECG cable snap connector 160 serves as the connective junction between the prior-described ECG module 120, base electrode 140 and remote electrodes 180 and 182. Exchangeable electrode and ECG cable snap connector 160 is shown clipped to the base ECG electrode snap connector. ECG module 120 is in turn attached to exchangeable electrode and ECG cable snap connector 160 such that the base ECG electrode holds both components in place. Exchangeable electrode and ECG cable snap connector 160 further comprises plug-in sockets which receive the lead wire connectors from remote ECG electrodes 180 and 182.

Exchangeable electrode and ECG cable snap connector 160 includes internal circuit traces and interface contacts. The circuitry passes signals from the base and remote ECG electrodes to the ECG module. The assembled components thus function together as an ambulatory ECG monitor/recorder. The assembly is sized to be worn under a patient's clothing, allowing the patient full mobility while in use.

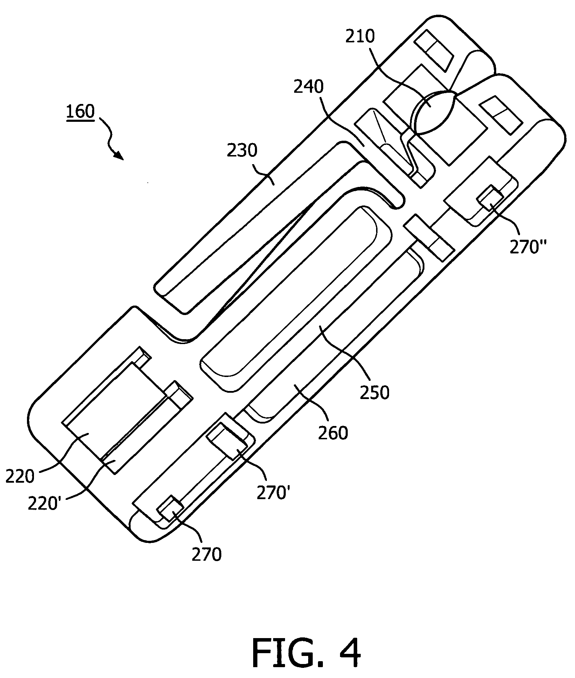

FIGURE 3a is a more detailed view showing the interaction between ECG module 120 and exchangeable electrode and ECG cable snap connector 160. ECG module 120 comprises a plurality of module charging/communication pins 340. Pins 340 are disposed to electrically mate with corresponding interconnect contacts 240 (shown in FIGURE 4) on the exchangeable electrode and ECG cable snap connector 160. ECG module 120 further comprises a module mount tongue 310 which is disposed to mate with a corresponding interconnect receiver 260 on the exchangeable electrode and ECG cable snap connector 160. A module mount tongue hook 320 located on one side of module mount tongue 310 is disposed to mate with an interconnect catch 250 on the exchangeable electrode and ECG cable snap connector 160 to hold the components securely together. Preferably, the hook 320 and catch 250 are designed to snap together for assembly. The catch 250 surface is flexible and can be flexed in order to disengage from hook 320 for disassembly. Alternatively, the components may be disengaged using a small tool such as a coin or screwdriver that may be inserted into the catch 250 slot to release hook 320.

FIGURE 3a also illustrates the exchangeable electrode and ECG cable snap connector 160 electrode connections. Shown here are plug-in lead wire sockets 220, 220' for receiving lead wire connectors from remote ECG electrodes 180 and 182. Also shown is the mounting clip 210 for physically and electrically securing exchangeable electrode and ECG cable snap connector 160 to base ECG electrode 140.

FIGURE 3b illustrates the assembly of exchangeable electrode and ECG cable snap connector 160, ECG module 120 and base ECG electrode 140. Exchangeable electrode and ECG cable snap connector 160 mounting clip 210 is arranged to be clipped to a corresponding snap connector 142 disposed on base ECG electrode 140. Engagement of clip 210 to connector 142 is accomplished by means of pressing clip spring arm 230, which opens clip 210 slightly around clip pivot 240. When clip spring 230 is release, clip 210 engages around connector 142 with sufficient force to ensure continuous electrical contact and to hold the connected exchangeable electrode and ECG cable snap connector 160 and ECG module 120 in place on the patient.

A preferred method of deploying the ECG monitor assembly for use begins by applying the base ECG electrode 140 and the remote ECG electrodes 180 and 182 to the patient. Application is achieved using an adhesive conductive gel which conducts electrical signals such as ECG from the patient to the ECG electrode. The gel serves also to hold the electrode in place. The user then clips the exchangeable electrode and ECG cable snap connector 160 to the base ECG electrode by depressing the clip spring arm 230, fitting the mounting clip 210 over the ECG electrode snap connector 142, and then releasing the clip spring arm 230. ECG module 120 is fitted to exchangeable electrode and ECG cable snap connector 160 by inserting module mount tongue 320 into the interconnect receiver 260 until the module mount tongue hook 320 snaps into place over interconnect catch 250. The user plugs the remote ECG electrode lead wire connectors into lead wire sockets 220, 220' to complete the assembly. The user activates ECG module 120, either locally or remotely, to begin the recording/monitoring operation.

The aforedescribed invention offers several important advantages over the prior art. The electrode and ECG cable snap connector 160 preferably contains all of the ECG monitor system's hard-to-clean female connections, such as the ECG lead wire sockets, the base electrode mounting clip 210, and the the interconnect receiver 260. By locating all of the female connections on the electrode and ECG cable snap connector 160, cleaning of the associated ECG module 120 and lead wires for remote ECG electrodes 180, 182 is greatly simplified. Electrode and ECG cable snap connector 160, by having no active electronics or power supply circuitry, is on the other hand inexpensive enough to be discarded after use as an alternative to cleaning. Thus, the electrode and ECG cable snap connector 160 reduces the cost and effort of using an ECG monitor assembly, while simultaneously reducing the risk of cross-contamination or infection.

An implementation of the invention is also more robust during repeated use. Wear surfaces for all of the electrical connections in the ECG monitor assembly are located in the electrode and ECG cable snap connector 160. The electrical contact pins 340 located on the ECG module 120 are not subject to wear by means of the secure connection with the electrode and ECG cable snap connector 160 contacts. Thus, the typically expensive ECG module 120 does not have to be discarded when electrical noise generated from worn-down electrical contact surfaces becomes excessive. Electrode and ECG cable snap connector 160 is discarded or replaced instead, saving substantial expense.

The present invention is also more gentle on the wearer. Because the electrode and ECG cable snap connector 160 can be clipped on to the base ECG electrode prior to the connection of the ECG module and other lead wires, the need for snap-pressing the entire assembly onto the base electrode at once is alleviated. This pressure on the patient's skin is sometimes painful, and the inventive method avoids the need to apply it.

FIGURE 4 is a detailed view of exchangeable electrode and ECG cable snap connector 160. Exchangeable electrode and ECG cable snap connector 160 comprises an elongated case which has connecting features on all but the outwardly-facing face. ECG electrode mounting clip 210 is disposed at one end of the case. Clip 210 is preferably split at the case end side such that it may be opened to receive the male snap connector 142 of the base ECG electrode. The clip 210 dimensions are such that when closed, clip 210 fits snugly around the periphery of male snap connector 142.

Clip spring arm 230 is disposed on an elongated side of the case. At rest, clip spring arm 230 is preferably flush with the case profile to minimize the likelihood of catching on the patient's clothing or other adjacent item. Clip spring arm 230 is pressed to operably open mounting clip 210 by causing an outside portion of clip 210 to rotate about clip pivot 240.

ECG lead wire sockets 220, 220' are disposed at the end of the case opposite mounting clip 210. The sockets 220, 220' are preferably dimensioned to receive standard ECG lead wire plugs. ECG module interconnect receiver 260 is disposed on the elongated side of the case opposite clip spring arm 230. Receiver 260 is dimensioned to receive ECG module mount tongue 310, and is preferably shaped such that the tongue must be inserted in the correct orientation. Receiver 260 further comprises interconnect catch 250 for securely mating with ECG module mount tongue hook 320.

Catch 250 is generally bar-shaped and is sized to be relatively flexible. When flexed through bending or gentle prying, catch 250 will deflect to release hook 320 and allow disengagement of the exchangeable electrode and ECG cable snap connector 160 from ECG module 120.

Exchangeable electrode and ECG cable snap connector 160 further comprises interconnect contacts 270, 270', 270" disposed on the elongated side of the case. The interconnect contacts are configured to electrically connect to corresponding contact pins on ECG module 120 when the components are joined together. The interconnect contacts are preferably spring-loaded to ensure good electrical contact with the ECG module pins when the parts are engaged.

Not shown in FIGURE 4 is an internal electrical circuit which electrically connects the ECG electrode mounting clip 210 and the ECG lead wire sockets 220, 220' to one of interconnect contacts 270, 270' or 270". Thus, when the ECG monitor assembly is complete, circuit paths are completed between each electrode and the ECG monitor 120 via exchangeable electrode and ECG cable snap connector 160.

The exchangeable electrode and ECG cable snap connector 160 case is comprised preferably of a material which is resistant to moisture, is rugged, and is inexpensive to manufacture. A preferred material for the case is a polycarbonate polymer, which may be molded in a single piece with the contacts and internal circuitry. The case is sized to fit compactly between ECG module 120 and base ECG electrode 140, and to be slim enough to be unobtrusive through the patient's clothing. Preferably, the exchangeable electrode and ECG cable snap connector 160 case is about 6 cm long by 2 cm wide by 1 cm in height.

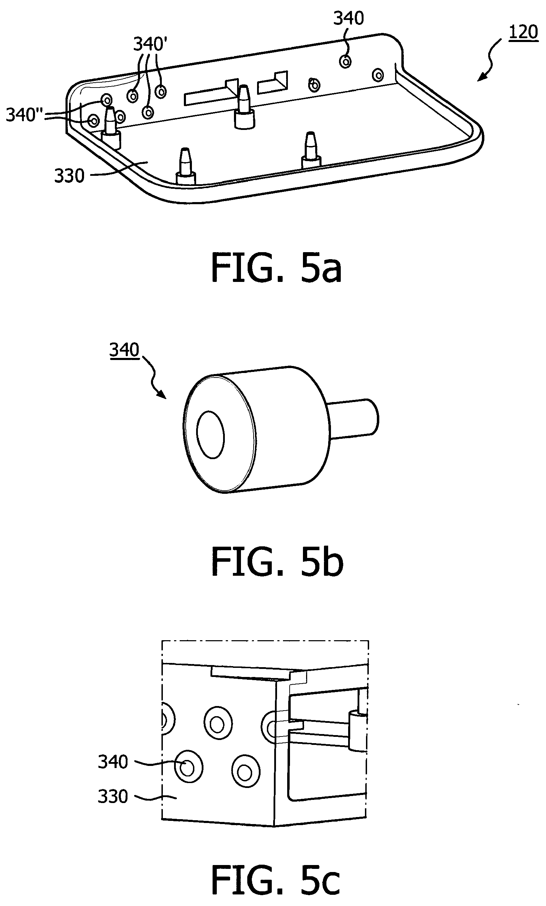

FIGUREs 5a and 5b show the ECG module 120 features which electrically interface with the exchangeable electrode and ECG cable snap connector 160. FIGURE 5a illustrates ECG module base 330, preferably of polymer material, with module charging/communication pins 340, 340', 340" molded into one wall. Pins 340, 340', 340" function as interface electrical contacts with corresponding interconnect contacts 270, 270', 270" on the exchangeable electrode and ECG cable snap connector 160. The pins are internally connected to the ECG module processing and storage circuitry not shown in this view.

FIGURE 5b is a detail view of one of charging/communication pins 340, 340', 340". Pin 340 is preferably plated with a conductive metal such as gold to facilitate good electrical contact. The pin 340 external contact surface is shaped slightly convex, such that the surface protrudes slightly from the module base 330, as shown in the detail view of FIGURE 5c. This feature reduces the difficulty of cleaning ECG module 120.

Other variations within the scope of the aforedescribed invention will readily occur to those skilled in the art. For instance, the particular dimensions of the exchangeable electrode and ECG cable snap connector may differ from the preferred size within the scope of the invention. The location and number of interface contacts may similarly differ within the scope of the invention.

## Claims

1. An exchangeable electrode and ECG cable snap connector unit (160) comprising:
means for mounting to a male electrical connector of a base ECG electrode;
at least one plug-in socket for receiving a lead wire connector of a remote ECG electrode (180) having a lead wire and said lead wire connector;
an interconnect receiver (260) for removably mating with a mount tongue of an ECG module;
an interconnect contact (270) for electrically contacting a charging/communication pin (340) of the ECG module; and
circuitry for electrically connecting the ECG module, base ECG electrode and remote ECG electrode.

2. The exchangeable electrode and ECG cable snap connector unit (160) of claim 1, wherein the means for mounting to a male electrical connector of a base ECG electrode comprise a mounting clip (210) for removably connecting to the base ECG electrode male electrical connector.

3. The exchangeable electrode and ECG cable snap connector unit (160) of claim 2, the monitoring clip further comprising a spring arm for removably attaching the mounting clip to the base ECG electrode male electrical connector.

4. The exchangeable electrode and ECG cable snap connector unit (160) of claim 3, further comprising further comprising a clip pivot for operably attaching the mounting clip spring arm to the ECG electrode mounting clip.

5. The exchangeable electrode and ECG cable snap connector unit (160) of claim 3, further comprising a case, wherein:
the ECG electrode mounting clip (210) is disposed at a first end of the case;
at least one plug-in socket comprises an ECG lead wire socket (220) disposed at a second end of the case;
the interconnect receiver is disposed on a first side of the case; and
the mounting clip spring arm is disposed on a second side of the case.

6. The exchangeable electrode and ECG cable snap connector unit (160) of claim 5, wherein the interconnect contact (270) comprises at least two interconnect contacts disposed on the first side of the case, wherein the two interconnect contacts are configured to electrically connect to corresponding contact pins on the ECG module.

7. The exchangeable electrode and ECG cable snap connector unit (160) of claim 6, wherein the circuitry for electrically connecting the ECG module, base ECG electrode and remote ECG electrode comprises an electrical circuit electrically connecting each of the ECG electrode mounting clip (210) and the ECG lead wire socket (220) to one of the two interconnect contacts.

8. The exchangeable electrode and ECG cable snap connector unit (160) of claim 7, wherein the interconnect contacts are spring-loaded.

9. The exchangeable electrode and ECG cable snap connector unit (160) of claim 5, further comprising an interconnect catch disposed on the first side of the case, the interconnect catch configured to secure an ECG module mount tongue hook (320).

10. A body-worn ECG monitor assembly (100), comprising:
a base ECG electrode (140) with a male electrical connector;
a remote ECG electrode (180) having a lead wire and lead wire connector;
an ECG module (120) comprising a mount tongue and a charging/communication pin (340); and
an exchangeable electrode and ECG cable snap connector (160) according to any of claims 1-9, wherein: the male electrical connector is electrically connected to the means for mounting to said male electrical connector;
the lead wire connector is electrically connected to the a one of said at least one plug-in socket,
the mount tongue is removably mated with the interconnect receiver; and
the charging/communication pin (340) is disposed in electrical contact with said interconnect contact (270).

11. A method of assembling an ambulatory ECG monitor comprising the steps of:
applying a base ECG electrode to a patient's torso;
connecting an exchangeable electrode and ECG cable snap connector according to any of claims 1-9 to the base ECG electrode;
attaching an ECG module to the exchangeable electrode and ECG cable snap connector after the connecting step;
adhering a remote ECG electrode to a patient's torso; and
plugging a lead wire connector from the remote ECG electrode into a socket in the exchangeable electrode and ECG cable snap connector.

12. The method of claim 11, further comprising the steps of:
collecting patient ECG information from the assembled ambulatory ECG monitor after the plugging step;
disconnecting the ECG module and the lead wire connector from the exchangeable electrode and ECG cable snap connector after the collecting step;
detaching the exchangeable electrode and ECG cable snap connector from the base ECG electrode; and
connecting a second exchangeable electrode and ECG cable snap connector to the base ECG electrode.

13. The method of claim 12, wherein the detaching step further comprises discarding the exchangeable electrode and ECG cable snap connector.

## Patentansprüche

1. Austauschbare Elektroden- und EKG-Kabel-Schnappverbindungseinheit (160), die Folgendes umfasst:
Mittel zum Montieren an einem männlichen elektrischen Steckverbinder einer Basis-EKG-Elektrode;
mindestens eine Einsteckbuchse zum Aufnehmen eines Ableitungssteckverbinders einer abgesetzten EKG-Elektrode (180) mit einer Ableitung und dem genannten Ableitungssteckverbinder;
einen Verbindungsaufnahme (260) zum lösbaren Zusammenführen mit einer Montagelasche eines EKG-Moduls;
einen Verbindungskontakt (270) zum elektrischen Kontaktieren eines Lade-/Kommunikationsstifts (340) des EKG-Moduls; und
Schaltungen zum elektrischen Verbinden des EKG-Moduls, der Basis-EKG-Elektrode und der abgesetzten EKG-Elektrode.

2. Austauschbare Elektroden- und EKG-Kabel-Schnappverbindungseinheit (160) nach Anspruch 1, wobei die Mittel zum Montieren an einem männlichen elektrischen Steckverbinder einer Basis-EKG-Elektrode einen Montageclip (210) umfassen, um eine lösbare Verbindung mit dem männlichen elektrischen Steckverbinder der Basis-EKG-Elektrode herzustellen.

3. Austauschbare Elektroden- und EKG-Kabel-Schnappverbindungseinheit (160) nach Anspruch 2, wobei der Montageclip weiterhin einen Federarm umfasst, um den Montageclip abnehmbar an dem männlichen elektrischen Steckverbinder der Basis-EKG-Elektrode anzubringen.

4. Austauschbare Elektroden- und EKG-Kabel-Schnappverbindungseinheit (160) nach Anspruch 3, weiterhin umfassend einen Clipdrehzapfen zum betriebsfähigen Anbringen des Montageclip-Federarms an dem Montageclip der EKG-Elektrode.

5. Austauschbare Elektroden- und EKG-Kabel-Schnappverbindungseinheit (160) nach Anspruch 3, weiterhin umfassend ein Gehäuse, wobei:
der Montageclip (210) der EKG-Elektrode an einem ersten Ende des Gehäuses angeordnet ist;
mindestens eine Einsteckbuchse eine EKG-Ableitungsbuchse (220) umfasst, die an einem zweiten Ende des Gehäuses angeordnet ist;
die Verbindungsaufnahme an einer ersten Seite des Gehäuses angeordnet ist; und
der Montageclip-Federarm an einer zweiten Seite des Gehäuses angeordnet ist.

6. Austauschbare Elektroden- und EKG-Kabel-Schnappverbindungseinheit (160) nach Anspruch 5, wobei der Verbindungskontakt (270) mindestens zwei Verbindungskontakte umfasst, die an der ersten Seite des Gehäuses angeordnet sind, wobei die beiden Verbindungskontakte konfiguriert sind, um eine elektrische Verbindung zu entsprechenden Kontaktstiften am EKG-Modul herzustellen.

7. Austauschbare Elektroden- und EKG-Kabel-Schnappverbindungseinheit (160) nach Anspruch 6, wobei die Schaltung zum elektrischen Verbinden des EKG-Moduls, der Basis-EKG-Elektrode und der abgesetzten EKG-Elektrode einen elektrischen Schaltkreis umfasst, der jeden von dem EKG-Elektroden-Montageclip (210) und der EKG-Ableitungsbuchse (220) mit einem der beiden Verbindungskontakte verbindet.

8. Austauschbare Elektroden- und EKG-Kabel-Schnappverbindungseinheit (160) nach Anspruch 7, wobei die Verbindungskontakte federbelastet sind.

9. Austauschbare Elektroden- und EKG-Kabel-Schnappverbindungseinheit (160) nach Anspruch 5, weiterhin umfassend einen Verbindungsschnappverschluss, der an der ersten Seite des Gehäuses angeordnet ist, wobei der Verbindungsschnappverschluss konfiguriert ist, um einen EKG-Modul-Montagelaschenhaken (320) zu sichern.

10. Am Körper getragene EKG-Überwachungsbaugruppe (100), die Folgendes umfasst:
eine Basis-EKG-Elektrode (140) mit einem männlichen elektrischen Steckverbinder;
eine abgesetzte EKG-Elektrode (180) mit einer Ableitung und einem Ableitungssteckverbinder;
ein EKG-Modul (120) umfassend eine Montagelasche und einen Lade-/Kommunikationsstift (340); und
einen austauschbaren Elektroden- und EKG-Kabel-Schnappverbinder (160) nach einem der Ansprüche 1 bis 9, wobei: der männliche elektrische Steckverbinder elektrisch mit dem Mittel zum Montieren an dem genannten männlichen elektrischen Steckverbinder verbunden ist;
der Ableitungssteckverbinder elektrisch mit einer von mindestens einer Einsteckbuchse verbunden ist;
die Montagelasche lösbar mit der Verbindungsaufnahme zusammengeführt ist; und
der Lade-/Kommunikationsstift (340) in elektrischem Kontakt mit dem genannten Verbindungskontakt (270) angeordnet ist.

11. Verfahren zum Zusammenbauen eines ambulatorischen EKG-Monitors, das die folgenden Schritte umfasst:
Anbringen einer Basis-EKG-Elektrode an dem Rumpf eines Patienten;
Verbinden eines austauschbaren Elektroden- und EKG-Kabel-Schnappverbinders nach einem der Ansprüche 1 bis 9 mit der Basis-EKG-Elektrode;
Anbringen eines EKG-Moduls an dem austauschbaren Elektroden- und EKG-Kabel-Schnappverbinder im Anschluss an den Schritt des Verbindens;
Aufkleben einer abgesetzten EKG-Elektrode auf den Rumpf eines Patienten; und
Einstecken eines Ableitungssteckverbinders von der abgesetzten EKG-Elektrode in eine Buchse in dem austauschbaren Elektroden- und EKG-Kabel-Schnappverbinder.

12. Verfahren nach Anspruch 11, das weiterhin die folgenden Schritte umfasst:
Erfassen von Patienten-EKG-Informationen von dem zusammengebauten ambulatorischen EKG-Monitor im Anschluss an den Schritt des Einsteckens;
Trennen des EKG-Moduls und des Ableitungssteckverbinders von dem austauschbaren Elektroden- und EKG-Kabel-Schnappverbinders nach dem Schritt des Erfassens;
Abnehmen des austauschbaren Elektroden- und EKG-Kabel-Schnappverbinders von der Basis-EKG-Elektrode; und
Verbinden eines zweiten austauschbaren Elektroden- und EKG-Kabel-Schnappverbinders mit der Basis-EKG-Elektrode.

13. Verfahren nach Anspruch 12, wobei der Schritt des Abnehmens weiterhin das Wegwerfen des austauschbaren Elektroden- und EKG-Kabel-Schnappverbinders umfasst.

## Revendications

1. Unité de connecteur à enclenchement de câble ECG et d'électrode échangeable (160) comprenant :
un moyen destiné à être sur un connecteur électrique mâle d'une électrode ECG de base ;
au moins une douille enfichable pour recevoir un connecteur de fil de sortie d'une électrode ECG distante (180) ayant un fil de sortie et ledit connecteur de fil de sortie ;
un récepteur d'interconnexion (260) pour s'accoupler de manière amovible avec une languette de montage d'un module ECG ;
un contact d'interconnexion (270) pour entrer en contact électrique avec une broche de charge/communication (340) du module ECG ; et
un ensemble de circuits pour entrer en contact électrique avec le module ECG, l'électrode ECG de base et l'électrode ECG distante.

2. Unité de connecteur à enclenchement de câble ECG et d'électrode échangeable (160) selon la revendication 1, dans laquelle le moyen destiné à être monté sur un connecteur électrique mâle d'une électrode ECG de base comprend un clip de montage (210) pour se connecter de manière amovible au connecteur électrique mâle d'électrode ECG de base.

3. Unité de connecteur à enclenchement de câble ECG et d'électrode échangeable (160) selon la revendication 2, le clip de montage comprenant en outre un bras de ressort pour attacher de manière amovible le clip de montage au connecteur électrique mâle d'électrode ECG de base.

4. Unité de connecteur à enclenchement de câble ECG et d'électrode échangeable (160) selon la revendication 3, comprenant en outre un pivot de clip pour attacher fonctionnellement le bras de ressort du clip de montage au clip de montage d'électrode ECG.

5. Unité de connecteur à enclenchement de câble ECG et d'électrode échangeable (160) selon la revendication 3, comprenant en outre un boîtier, dans laquelle :
le clip de montage d'électrode ECG (210) est disposé au niveau d'une première extrémité du boîtier ;
au moins une douille enfichable comprend une douille de fil de sortie ECG (220) disposée au niveau d'une seconde extrémité du boîtier ;
le récepteur d'interconnexion est disposé sur un premier côté du boîtier ; et
le bras de ressort du clip de montage est disposé sur un deuxième côté du boîtier.

6. Unité de connecteur à enclenchement de câble ECG et d'électrode échangeable (160) selon la revendication 5, dans laquelle le contact d'interconnexion (270) comprend au moins deux contacts d'interconnexion disposés sur le premier côté du boîtier, dans laquelle les deux contacts d'interconnexion sont configurés pour se connecter électriquement à des broches de contact correspondantes sur le module ECG.

7. Unité de connecteur à enclenchement de câble ECG et d'électrode échangeable (160) selon la revendication 6, dans laquelle l'ensemble de circuits pour connecter électriquement le module ECG, l'électrode ECG de base et l'électrode ECG distante comprend un circuit électrique connectant électriquement chacun du clip de montage d'électrode ECG (210) et de la douille de fil de sortie ECG (220) à l'un des deux contacts d'interconnexion.

8. Unité de connecteur à enclenchement de câble ECG et d'électrode échangeable (160) selon la revendication 7, dans laquelle les contacts d'interconnexion sont à ressort.

9. Unité de connecteur à enclenchement de câble ECG et d'électrode échangeable (160) selon la revendication 5, comprenant en outre un cran d'interconnexion disposé sur le premier côté du boîtier, le cran d'interconnexion étant configuré pour fixer un crochet de languette de montage de module ECG (320).

10. Ensemble de contrôle ECG porté sur le corps (100), comprenant :
une électrode ECG mâle (140) avec un connecteur électrique mâle ;
une électrode ECG distante (180) ayant un fil de sortie et un connecteur de fil de sortie ;
un module ECG (120) comprenant une languette de montage et une broche de charge/communication (340) ; et
un connecteur à enclenchement de câble ECG et d'électrode échangeable (160) selon l'une quelconque des revendications 1-9, dans lequel : le connecteur électrique mâle est électriquement connecté au moyen destiné à être monté sur ledit connecteur électrique mâle ;
le connecteur de fil de sortie est électriquement connecté à l'une de ladite au moins une douille enfichable ;
la languette de montage s'accouple de manière amovible avec le récepteur d'interconnexion ; et
la broche de charge/communication (340) est disposée en contact électrique avec ledit contact d'interconnexion (270).

11. Procédé d'assemblage d'un dispositif de contrôle ECG ambulatoire comprenant les étapes consistant à :
appliquer une électrode ECG de base sur le torse d'un patient ;
connecter un connecteur à enclenchement de câble ECG et d'électrode échangeable selon l'une quelconque des revendications 1-9 à l'électrode ECG de base ;
attacher un module ECG au connecteur à enclenchement de câble ECG et d'électrode échangeable après l'étape de connexion ;
faire adhérer une électrode ECG distante au torse d'un patient ; et
brancher un connecteur de fil de sortie depuis l'électrode ECG distante dans une douille du connecteur à enclenchement de câble ECG et d'électrode échangeable.

12. Procédé selon la revendication 11, comprenant en outre les étapes consistant à :
collecter des informations ECG du patient à partir du dispositif de contrôle ECG ambulatoire assemblé après l'étape de branchement ;
déconnecter le module ECG et le connecteur de fil de sortie du connecteur à enclenchement de câble ECG et d'électrode échangeable après l'étape de collecte ;
détacher le connecteur à enclenchement de câble ECG et d'électrode échangeable de l'électrode ECG de base ; et
connecter un deuxième connecteur à enclenchement de câble ECG et d'électrode échangeable à l'électrode ECG de base.

13. Procédé selon la revendication 12, dans lequel l'étape de détachement comprend en outre le rejet du connecteur à enclenchement de câble ECG et d'électrode échangeable.
